(19) Europäisches Patentamt
European Patent Office
Office européen des brevets

(11) Veröffentlichungsnummer: **0 126 313**
**B1**

(12) # EUROPÄISCHE PATENTSCHRIFT

(45) Veröffentlichungstag der Patentschrift: **08.08.90**

(51) Int. Cl.$^5$: **A 61 K 39/39**

(21) Anmeldenummer: **84104547.9**

(22) Anmeldetag: **21.04.84**

(54) **Verwendung des Diterpen-Derivates Forskolin zur Immunstimulation.**

(30) Priorität: **26.04.83 DE 3314999**

(43) Veröffentlichungstag der Anmeldung:
**28.11.84 Patentblatt 84/48**

(45) Bekanntmachung des Hinweises auf die Patenterteilung:
**08.08.90 Patentblatt 90/32**

(84) Benannte Vertragsstaaten:
**AT BE CH DE FR GB IT LI LU NL SE**

(56) Entgegenhaltungen:
**ARZNEIM.-FORSCH., Band 31, Nr. 8, 1981, Seiten 1248-1250; H. METZGER et al.: "The positive inotropic-acting Forskolin, a potent adenylatecyclase activator"**

**IMMUNOSTIMULATION, Ed. L. Chedid et al., 1982, Seiten 33-48, Springer-Verlag, Berlin, DE; J.W. HADDEN: "The immunopharmacology of immunotherapy"**

(73) Patentinhaber: **BEHRINGWERKE Aktiengesellschaft
Postfach 1140
D-3550 Marburg 1 (DE)**
(73) Patentinhaber: **HOECHST AKTIENGESELLSCHAFT
Postfach 80 03 20
D-6230 Frankfurt am Main 80 (DE)**

(72) Erfinder: **Schorlemmer, Hans-Ulrich, Dr.
An der Neuen Schule 14
D-3550 Marburg (DE)**
Erfinder: **Dickneite, Gerhard, Dr.
Zum neuen Hieb 31
D-3550 Marburg 15 (DE)**
Erfinder: **Sedlacek, Hans-Harald, Dr.
Sonnenhang 3
D-3550 Marburg (DE)**
Erfinder: **DE Souza, Noel John, Dr.
702 Avanti, Central Avenue
Santa Cruz, Bombay 400 054 (IN)**
Erfinder: **Dohadwalla, Alihussein Nomanbhai, Dr.
139C, Cumballa Hill
Bombay 400 036 (IN)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 126 313 B1

(56) Entgegenhaltungen:
ANN. N.Y. ACAD. SCI., Band 332, 1979, Seiten 255-261; C.W. PARKER.: "Role of cyclic nucleotides in regulating lymphocytes"

CHEMICAL ABSTRACTS, Band 99, Nr. 5, 1. August 1983, Seite 2, Zusammenfassung Nr. 32604k, Columbus, Ohio, US; N.J. DE SOUZA et al.: "Forskolin: a labdane diterpenoid with antihypertensive, positive inotropic, platelet aggregation inhibitory, and adenylate cyclase activating properties"

(74) Vertreter: Meyer-Dulheuer, Karl-Hermann, Dr. et al
HOECHST Aktiengesellschaft Postfach 3540
D-6200 Wiesbaden (DE)

**Beschreibung**

Die Erfindung betrifft die Verwendung des pharmakologisch wirksamen Diterpen-Derivates Forskolin zur Stimulierung der immunologischen Reaktivität bei Säugern.

Forskolin ist in der deutschen Offenlegungsschrift 25 57 784 beschrieben und dafür die folgende Struktur angegeben:

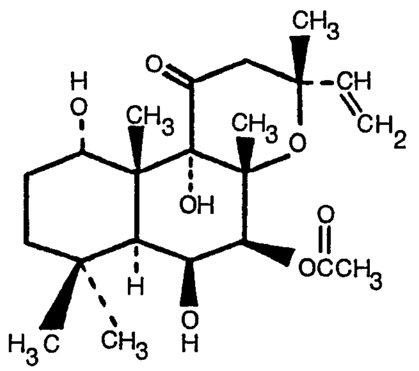

(1α,6β,9α-trihydroxy-7β-acetoxy-8,13-epoxy-labol-14-en-11-one)

Forskolin wurde aus Coleus forskohlii isoliert und wirkt positiv inotrop und blutdrucksenkend. Es aktiviert membrangebundene Adenylatcyclase in allen bisher untersuchten Säugetiergeweben, und dies führt zu einer Erhöhung des zellulären cAMP-Spiegels.

Es ist bekannt, daß die Abwehrmechanismen des Organismus, die kurz als humorale Immunität und zelluläre Immunität bezeichnet werden, zusammenwirken, um Fremdkörper, die schädlich sein können, zu eliminieren, vornehmlich Mikroorganismen oder neoplastische Zellen.

Immunologische Untersuchungen ergaben, daß Zusammenhänge zwischen der natürlichen oder durch äußere Faktoren provozierten Abnahme der immunologischen Aktivität und der Zunahme der Injektions- oder Tumorgefahr bestehen. Daneben entstehen andere Krankheiten durch Veränderungen der Funktionen des Immunsystems, wie beispielsweise Autoimmunkrankheiten oder durch Immunkomplexe hervorgerufene Erkrankungen, Intoxikationen, Septikämien und dergleichen. Man sucht deshalb nach Immunstimulantien, d.h. nach Subtanzen, die imstande sind, die immunolgische Aktivität des Empfängers zu verändern, vorzugsweise zu erhöhen. Beispiele, die hinsichtlich der Stimulation der Immunität geprüft wurden, sind BCG und C. parvum, ferner die Extrakte des M. tuberculosis und der Brucellen.

Diese Substanzen erzeugen jedoch in den Konzentrationen, wie sie zur Anwendung kommen, deutliche Nebenwirkungen wie z.B. in verschiedenem Ausmaß lokale Granulome. Die Unkenntnis der genauen Natur der Substanzen erschweren eine systematische Untersuchung mit guter Reproduzierbarkeit der klinischen Ergebnisse. Besser ist in diesem Zusammenhang die neue Generation der Immunstimulantien mit chemisch genau definierten Substanzen bei geringer Toxizität wie z.B. Bestatin.

Es wurde nun überraschend gefunden, daß Forskolin die Immunreaktion bei warmblütigen Säugern in einer Konzentration von weniger als 20 mg/kg Körpergewicht stimuliert, ohne dabei toxische Nebenwirkungen zu zeigen, und damit für die Behandlung von Krankheiten des Immunsystems geeignet ist. Diese Verbindung erhöht die Produktion von Antikörpern und verstärkt die zelluläre Immunität und ermöglicht damit eine immunologische Aktivität bei solchen Patienten weiderherzustellen, deren immunologische Abwehr auf natürliche Weise oder durch irgendeine Provokation herabgemindert ist.

Gegenstand der Erfindung ist die Verwendung von Forskolin als Immunpotentiator.

Die wirksame immunstimulierende Menge liegt im Bereich von 2,5 bis 20 mg/kg Körpergewicht bei parenteraler Verabreichung.

Für die parenterale, speziell intravenöse Verabreichung kommen Lösungen oder Suspensionen des Wirkstoffes in einem pharmazeutisch verträglichen Vektor, vorzugsweise Pflanzenöl, wie z.B. Erdnußöl oder Sesamöl sowie alkoholische Lösungen des Wirkstoffes, z.B. in Äthanol, Propandiol oder Glycerin oder in Gemischen der vorgenannten Lösungsmittel in Frage.

Die entsprechend der Erfindung verwendete Verbindung besitzt in den hierfür wirksamen Konzentrationsbereichen eine nur geringe Toxizität und führt nicht zur lokalen Granulombildung.

Im folgenden wird die Einwirkung der Verbindung auf die Immunantwort der Maus und ihre immunstimulierende Aktivität in verschiedenen in vitro und in vivo-Standard-testmethoden, die bekanntermaßen für die Beurteilung von Immunstimulantien herangezogen werden, beispielhaft erläutert.

Beispiel 1

Wirkung der Substanz auf die Antikörperantwort gegen Escherichia coli-Totkeime.

Es wurden Gruppen von 5 weiblichen NMRI-Mäusen mit einem Gewicht von 18—20 g intravenös jeweils $10^8$ hitzeabgetötete E.coli-Bakterien pro Tier verabreicht. Hiervon wurde einer Mäusegruppe als Kontrolle gleichzeitig physiologische Kochsalzlösung intravenös appliziert. Andere Grupen erhielten

gleichzeitig aber separat auf intravenösem Weg die Test-Substanz in verschiedenen Konzentrationen in physiologischer Kochsalzlösung, wobei die Dosen von 2,5 mg/kg, 5 mg/kg, 10 mg/kg auf 20 mg/kg gestaffelt anstiegen.

20 Tage nach der Injektion des Antigens und der verschiedenen Konzentrationen der Verbindung Forskolin wurde den Mäusen aus dem retroorbitalen Venengeflecht Blut entnommen und die Antikörper gegen E.coli in dem daraus gewonnenen Serum bestimmt. Die Bestimmung der Antikörper (IgG) wurde mit Hilfe der dem Fachmann bekannten ELISA-Technik durchgeführt, wobei als Antigen homologes Lipopolysaccharid von E.coli benutzt wurde. Die Höhe der photometrisch gemessenen Extinktionswerte ist ein Maß für die Menge der gebildeten Antikörper. Die Ergebnisse sind in der nachstehenden Tabelle 1 zusammengestellt.

TABELLE 1

Einfluß von Forskolin auf die Antikörperantwort bei Immunisierung von Mäusen mit Escherichia coli-Totkeim

| 1 × Applikation i.v. am Tag O | Antikörperantwort ($mE_{492\ nm}$ — ELISA-Test) |
|---|---|
| PBS | 1151 ± 311 |
| Forskolin 2,5 mg/kg | 1382 ± 324 |
| Forskolin 5 mg/kg | 1692 ± 192* |
| Forskolin 10 mg/kg | 1551 ± 332** |

Statistische Signifikanz (Student t-Test:* $p < 0{,}01$;** $p < 0{,}05$).

Aus Tabelle 1 ist zu ersehen, daß die Substanz in dem untersuchten Mäusemodell zu einer dosisabhängigen Stimulation der Antikörper-Antwort führt. Bei einer Dosis von 5 mg/kg wird eine maximale immunitätsstimulierende Wirkung für die Verbindung erreicht.

## Beispiel 2

Wirkung von Forskolin auf die zelluläre Immunantwort gegen Schafserythrozyten.

In diesem Beispiel wurden Mäuse analog der im Beispiel 1 beschriebenen Weise behandelt mit dem Unterschied, daß den Mäusen intravenös entweder $10^6$ oder $10^9$ rote Blutkörperchen vom Schaf pro Tier injiziert wurden. Schafserythrozyten gelten in der Immunologie als Standardprüf-substanz (Antigen) zur Erkennung der zellulären und humoralen Immunreaktion. Gleichzeitig wurde die erfindungsgemäße Substanz in den Konzentrationen 2,5 mg/kg, 5 mg/kg, 10 mg/kg und 20 mg/kg in physiologischer Kochsalzlösung intravenös appliziert. Nach 5 Tagen wurde allen Tieren jeweils $2 \times 10^8$ Schafserythrozyten in die Fußsohle injiziert und 24 Stunden später wurde die Schwellung des Fußes gemessen. Die Fußschwellung wird durch eine Hautreaktion vom verzögerten Typ (Delayed Type Hypersensitivity, DTH) ausgelöst und ist, wie dem Fachmann bekannt, ein Maß für die zelluläre Immunantwort (Collins, F M. und Mackaness, G.B., J. Immunol. (1968), *101*, 830—845). Die in der Tabelle 2 zusammengestellten Ergebnisse veranschaulichen, daß es durch die Verabfolgung des Forskolins sowohl nach Immunisierung mit $10^6$ als auch mit $10^9$

Schafserythrozyten zur Steigerung der zellulären Immunantwort kommt. Ein Maximum der Stimulation kann auch in diesem Experimentalansatz bei der Gabe von 5 mg/kg Substanz beobachtet werden.

TABELLE 2

Immunisiserung von Mäusen mit Schafserythrozyten-Wirkung von Forskolin auf die zelluläre Immunantwort (DTH-Reaktion)

| 1 × i.v. Applikation von | % Fußschwellung bei $10^6$ Erythrozyten |
|---|---|
| PBS | 16.0 |
| Forskolin 2,5 mg/kg | 18.6 |
| Forskolin 5 mg/kg | 21.5 |
| Forskolin 10 mg/kg | 17.1 |
| Forskolin 20 mg/kg | 23.2 |

Beispiel 3

Der Einfluß von Forskolin auf die Stimulation der unspezifischen Immunität — Aktivierung von mononukleären Phagozyten.

Hier wurde der Einfluß der erfindungsgemäß verwendeten Verbindung auf die Stimulation von Peritonealmakrophagen bei 6—8 Wochen alten NMRI-Mäusen untersucht. Mäuseweibchen erhielten auf parenteralem (intravenös und intraperitoneal) Wege die Prüfsubstanz in einer Dosis von 2,5 mg/kg, 5 mg/kg, 10 mg/kg und 20 mg/kg. Der Kontrollgruppe wurde gepufferte Kochsalzlösung verabreicht. 3 Tage nach den Injektionen wurden die Mäuse getötet und es wurden die Peritonealmakrophagen der Tiere auf ihren Aktivierungszustand hin untersucht. Als Maß der Makrophagenaktivierung wurde zum einen die Sekretion der lysosomalen Enzyme (β-Glucuronidase, β-Galactosidase, NAcetyl-β-D-glucosaminidase) bestimmt. Auf der anderen Seite konnte in den gleichen Makrophagenkulturen auch die Synthese von den Prostaglandinen E2 und $F2_{\alpha}$ mit Hilfe der RIA-Technik (wie sie dem Fachmann bekannt ist) untersucht werden. Die Höhe des oxidativen Stoffwechsels bei Makrophagen gilt als ein weiteres Maß für ihren Aktivierungszustand. Gemessen wird diese Aktivität unter Zuhilfenahme des Biolumaten durch Bestimmung der Chemolumineszenz.

Zu diesem Zweck wurden entweder in Petrischalen von 30 mm Durchmesser 3 × $10^6$ Makrophagen mit 1 ml TC199-Kulturmedium oder aber $10^6$ Makrophagen mit 100 µl Medium in Rundboden-Polyäthylen-Röhrchen (zur Bestimmung der Chemolumineszenz) bei 5% $CO_2$ und 37°C kultiviert. Nach einstündiger Bebrütung werden die Kulturen gewaschen, um schwimmende Zellen zu entfernen. die Chemolumineszenz (Röhrchenkultur) wurde dann direkt bestimmt, während die Petrischalen erneut 24 Stunden bei 37°C bebrütet wurden und danach die Enzym- und Prostaglandinaktivität in den Kulturen bestimmt wurde. Es wurden die nachstehenden Ergebnisse erzielt.

TABELLE 3

Wirkung von Forskolin auf die Makrophagenaktivität (Chemolumineszenz in RLE/15 Minuten*)

| 1 × Applikation von | intraperitoneal | intravenös |
|---|---|---|
| PBS | 1855 ± 59 | 1263 ± 37 |
| Forskolin 2,5 mg/kg | 6529 ± 74 | 3351 ± 42 |
| Forskolin 5 mg/kg | 17134 ± 142 | 10476 ± 98 |
| Forskolin 10 mg/kg | 24199 ± 295 | 18871 ± 211 |
| Forskolin 20 mg/kg | 38421 ± 232 | 26029 ± 145 |

RLE = Relative Lichteinheiten

Die parenterale Behandlung von NMRI-Mäusen mit Forskolin führt zu einer immunitäts-stimulierenden Wirkung. Der oxidative Stoffwechsel bei Makrophagen mit der Generierung von Sauerstoffradikalen und dem damit verbundenen meßbaren Licht ist deutlich erhöht. Bei Dosierungen von 2,5 mg/kg aufwärts kommt es zu einer dosisabhängigen Steigerung der Makrophagenaktivität.

Aus der Tabelle 4 ist zu entnehmen, daß Makrophagen von Kontrollmäusen nur geringe Mengen an lysosomalen Enzymen (β-Glucuronidase, β-Galactosidase, N-Acetyl-β-D-Glucosaminidase) in den Kulturüberstand abgeben. Mononukleäre Phagozyten von Mäusen, die parenteral mit der Verbindung 72 Stunden behandelt wurden, sezernieren die o.a. sauren Hydrolasen (β-Glu, β-Gal und N-Acetyl-Glu) deutlich mehr und weisen damit eine Dosiswirkungskurve auf, die bei allen gemessenen Enzymen eine Überlegenheit gegenüber den Kontrollen erkennen läßt. Es ist ersichtlich, daß Forskolin eine stimulierende Wirkung auf die Makrophagenaktivität besitzt und zur Erhöhung der Enzymfreisetzung beiträgt.

TABELLE 4

Einfluß der Prüfsubstanz auf die Enzymfreisetzung lysosomaler Hydrolasen von Maus-Peritonealmakrophagen

| 1 × i.p./i.v. Applikation | β-Glu mU/ml | β-Gal mU/ml | N-Acetyl-Glu mU/ml |
|---|---|---|---|
| PBS | 817/ 709 | 5866/ 4429 | 5426/3120 |
| Forskolin 2,5 mg/kg | 1088/ 924 | 9532/ 7537 | 748/4842 |
| Forskolin 5 mg/kg | 1305/1181 | 11418/ 9250 | 9076/5665 |
| Forskolin 10 mg/kg | 1487/1363 | 12358/10812 | 12479/7081 |
| Forskolin 20 mg/kg | 1669/1575 | 13867/12228 | 15812/9146 |

Die Ergebnisse in Tabelle 5 veranschaulichen den Effekt von Forskolin auf die Prostaglandinproduktion. Bei diesem Effekt ist die Menge (pg/ml) an synthetisiertem Prostaglandin E2 und $F2_{\alpha}$ im Kulturüberstand gemessen worden. die intravenöse Verabfolgung der Prüfsubstanz an die NMRI-Mäuse erhöhte signifikant je nach der Behandlungsdosis die Synthese on Progaglandinen bei Peritonealmakrophagen.

TABELLE 5

Der Effekt von Forskolin auf die Prostaglandinproduktion durch Mausmakrophagen

| 1 × i.v. Applikation von | $PGE_2$ (pg/ml) | $PFG_{2\alpha}$ (pg/ml) |
|---|---|---|
| PBS | 724 | 598 |
| Forskolin 2,5 mg/kg | 4.356 | 2.644 |
| Forskolin 5 mg/kg | 7.082 | 3.782 |
| Forskolin 10 mg/kg | 9.146 | 4.258 |
| Forskolin 20 mg/kg | 11.498 | 5.163 |

Die Ergebnisse aus den Tabellen 3, 4 und 5 zeigen deutlich, daß sich die Verbindung Forskolin bei den verschiedenen Dosen, die berücksichtigt wurde, als wirksam zur Erhöhung des immunologischen Potentials erwies, zu einer Stimulation der Makrophagenaktivität und damit zu einer Stimulation der unspezifischen Immunität führt.

Beispiel 4

Kinetik des Erscheinens aktivierter Makrophagen bei Forskolin behandelten Mäusen

In diesem Beispiel wurden Mäuse analog der im Beispiel 3 beschriebenen Weise mit 10 mg/kg der Prüfsubstanz behandelt. An den Tagen 3, 4, 5, 6, 10, 11, 12 und 13 nach Injektion wurden die Mäuse getötet und die Peritonealmakrophagen der Tiere auf ihre Enzymaktivität geprüft.

Die Ergebnisse sind in Tabelle 6 zusammengestellt.

TABELLE 6

Kinetik aktivierter Maus-Peritonealmakrophagen bei Forskolin-Behandlung. Bestimmung der %-Freisetzung von β-Glucuronidase

| Tage der Enzymbestimmung (β-Glu) | PBS | i.v.-Applikation Forskolin (20 mg/kg) |
|---|---|---|
| 3 | 8.4 ± 0.7% | 21.3 ± 1.6% |
| 4 | 9.7 ± 1.2% | 28.9 ± 1.3% |
| 5 | 9.2 ± 0.5% | 37.4 ± 1.5% |
| 6 | 10.6 ± 1.4% | 46.8 ± 1.9% |
| 10 | 9.5 ± 0.8% | 53.6 ± 2.2% |
| 11 | 11.3 ± 1.1% | 41.7 ± 1.6% |
| 12 | 10.9 ± 0.9% | 32.4 ± 1.5% |
| 13 | 9.8 ± 1.0% | 26.8 ± 1.1% |

Das Erscheinen aktivierter Makrophagen (hier bestimmt durch die %-Freisetzung des lysosomalen Enzyms β-Glucuronidase) ist recht schnell. Schon nach 3 Tagen sind die Makrophagen aktiviert, steigen noch stetig in ihrer Aktivität an, erreichen an den Tagen 6—10 ein Optimum, um danach wieder mit zunehmender Zeit nach der Forskolin-Injektion in ihrer Aktivität abzusinken. Im Gegensatz dazu zeigen Makrophagen von Kontrolltieren (PBS-Behandlung) zu keiner Zeit eine erhöhte Enzymaktivität.

Nach diesen Ergebnissen aus Tabelle 6 ist ersichtlich, daß 10 mg/kg Forskolin nach intravenöser Applikation bei Maüsen die unspezifische Immunität (Makrophagenaktivität) schon nach 3 Tagen stimuliert; das Optimum dieser Stimulation ist jedoch erst nach ca. 10 Tagen erreicht.

Zusammenfassend kann also festgestellt werden, daß Forskolin in verschiedenen in vitro und in vivo Standard-testmethoden, die algemein für die Beurteilung von Immunstimulantien herangezogen werden, imstande ist, die immunologische Aktivität des Empfängers zu erhöhen. Damit ist Forskolin auch in der Lage, die Abwehrmechanismen des Organismus zu aktivieren und kann zur Abnahme der Infektions- und Tumorgefahr beitragen.

**Patentanspruch**

Verwendung von Forskolin zur Herstellung eines Arzneimittels zur stimulierung der Immunreaktion.

**Revendication**

Utilisation de la forskoline pour la fabrication d'un médicament pour la stimulation de la réaction immunitaire.

**Claim**

The use of forskolin for the preparation of a medicament for stimulation of the immune response.